# EUROPEAN PATENT SPECIFICATION

(11) **EP 0 994 731 B1**
(45) Date of publication and mention of the grant of the patent: **18.12.2002**
(21) Application number: 98932272.2
(22) Date of filing: 03.07.1998
(51) Int. Cl.: A61L 2/24

(54) **CLEANING APPARATUS**
VORRICHTUNG ZUR REINIGUNG VON MEDIZINISCHEN GERÄTEN
APPAREIL DE NETTOYAGE

(30) Priority: 04.07.1997 GB 9714127
(43) Date of publication of application: 26.04.2000
(73) Proprietor: The University of Sheffield, Sheffield S10 2TE (GB)
(72) Inventor: ZALZALA, Ali, Mohammad, Sadik, Edinburgh EH10 5HT (GB); NICKLIN, Graham, Sheffield S31 8FS (GB)
(74) Representative: Stainthorpe, Vanessa J.
(86) International application number: GB9801764
(87) International publication number: WO99001165

(56) References cited:
- EP-A- 0 738 493
- US-A- 4 067 691

## Description

The present invention relates to cleaning apparatus for cleaning, for example, medical instruments.

DE 3516006 C1 discloses a washing and disinfecting machine for disinfecting medical instruments. The machine receives a tray loaded with instruments to be cleaned. Conventionally, as each type of instrument or tray full of such instruments may require a particular cleaning programme or use of prescribed cleaning detergents, a human operator would select the detergent or disinfectant and cleaning programme appropriate to the instruments to be cleaned and instigate the cleaning process accordingly.

However, a human operator may occasionally load an incorrect detergent and/or disinfectant or select an inappropriate cleaning programme resulting in medical instruments which have not been correctly cleaned. Clearly, this would result in an increased possibility of infection of a patient.

DE 3516006 C1 addressed this problem by removing the human element from the selection of the appropriate cleaning programme. The cleaning programme is determined by the tray itself. Any given tray is designed to hold only instruments of a particular type. The tray comprises means for automatically selecting or indicating the cleaning programme appropriate to the instruments for which the tray was designed to accommodate. This significantly reduces the risk of the incorrect cleaning programme being used for particular types of instrument.

Use of a plurality of cleaning apparatuses such as those described in DE 3516006 C1 still requires a significant degree of human interaction to load and unload the disinfection units. Furthermore, DE 3516006 C1 does not address the problem of inserting dirty articles into the disinfecting apparatus and removing clean articles from the same side of the disinfecting apparatus. This increases the risk of contamination of the clean articles by dirty articles. US-A- 4067691 also discloses a cleaning and sterilizing apparatus which is automated.

It is an object of the present invention to at least mitigate some of the problems associated with prior art cleaning apparatus.

Accordingly, the present invention provides apparatus for cleaning articles comprising a plurality of cleaning units each having a chamber for receiving a tray loaded with articles to be cleaned, said cleaning unit being arranged to clean said articles, a transport mechanism for loading and removing said tray of articles into and out of selectable ones of said chambers, and control means for automatically coordinating the operation of said plurality of cleaning units and said transport mechanism, wherein said control means comprises a master control processor and associated with each of the said cleaning units at least one slave processor for controlling the operation of said plurality of cleaning units in response to data received from the master control processor.

Advantageously, the cleaning apparatus of the present invention renders the process of cleaning surgical instruments less manpower intensive. As the delivery of the trays to individual washing or cleaning units is controlled, the overall productivity of the cleaning apparatus is increased.

Further, the apparatus of the present invention removes the requirement for human operators to load, unload and operate the washing machines. Consequently, an improved level of safety is provided.

Further, since an embodiment of the cleaning apparatus comprises a "dirty" side and a "clean" side, the risk of contamination of clean articles by dirty articles is significantly reduced.

The cleaning programmes executed by the slave processors are governed by the master controller or processor. This gives increased flexibility and, in the event of a change to any given cleaning programme, that change can be catered for centrally by changing the single copy the control software downloaded by the master processor rather than changing the software of each of the slave processors.

The doors of conventional disinfection or cleaning units are hinged at the along one of the vertical sides thereof. Such a door prevents the use of some form of fixed automated loading or extraction mechanism and, in the event of automation, would require extremely expensive tray bearing mobile units, robots or the like.

Suitably, the present invention provides cleaning apparatus wherein said chamber comprises at least a first slidably closable door via which the tray can be received into or removed from the chamber. Alternatively, the present invention provides a first hinged closable chamber door hingedly coupled to the cleaning unit along an upper horizontal edge thereof.

In order to improve the hygiene or to maintain the disinfected state, it is desirable to minimise or substantially reduce the possibility of contact between clean and dirty items.

Accordingly, an apparatus is provided further comprising a second slidably closable door via which the tray can be received into or removed from the chamber, preferably, said first and second slidably closable doors are disposed on substantially opposite sides of the cleaning apparatus.

An aspect of the present invention provides apparatus wherein said transport means comprises a conveyor for conveying trays loaded thereon to a first position for loading into said chamber. Preferably, said first position is selectable by said control means in response to at least one of a plurality of conditions, for example, one of said plurality of conditions is governed by a current utilisation status of one of a plurality of cleaning units. Alternatively, one of said plurality of conditions is governed by the remaining time until the end of the operation of a currently executing cleaning cycle of the cleaning unit.

A further aspect of the present invention provides apparatus wherein said transport mechanism comprises an input mechanism for correctly positioning the tray in front of a selectable one of said plurality of cleaning units for loading thereinto and an output mechanism for transporting said tray away from a respective cleaning unit. An embodiment is provided wherein said input and output transport mechanisms are disposed on substantially opposite sides of said apparatus.

Often disinfection or cleaning units comprise a limited number of preprogrammed cleaning programmes. Each programme may be suitable for a particular type of article to be cleaned. However, as cleaning techniques develop, preprogrammed cleaning programmes may become obsolete.

The cleaning environment including the potentially hazardous cleaning chemicals and temperatures may, in the event of the failure of a disinfection unit, result in injury to the operators of the units. Therefore, an embodiment of the present invention provides cleaning apparatus wherein said first control processor is remotely disposed, screened or physically separated from said cleaning units and said transport mechanism.

In the event of, for example, infection of a patient in a hospital, the source of the infection may be attributable to ineffectively disinfected instruments used in relation to or on that patient. The ineffectively disinfected instruments usually follow as a natural consequence of a failing or incorrectedly operated disinfection unit.

Suitably, an aspect of the present invention provides a cleaning apparatus wherein said control means further comprises means for collating and outputting, via a user interface, data related to the operating conditions of the cleaning units. Preferably, the operating or cleaning temperatures of the cleaning units are periodically measured and recorded during a cleaning cycle.

Embodiments of the present invention will now be described, by way of example only, with reference to the accompanying drawings in which:
figure 1 illustrates schematically cleaning apparatus in accordance with an embodiment for cleaning, for example, surgical instruments;
figure 2 illustrates two of the plurality of disinfecting units together with corresponding portions of the conveyors, loading and extraction mechanisms;
figures 3a to 3d show schematically an engagement/disengagement mechanism for removing a tray from a disinfecting chamber of a disinfecting unit;
figure 4 depicts schematically a circuit used in conjunction with capacitive sensors;
figure 5 shows circuitry for controlling the pneumatic systems of the cleaning apparatus;
figure 6 illustrates the pneumatic systems for controlling the position of carriages of the loading and extraction mechanism;
figure 7 shows the inter-processor communication architecture for coordinating and controlling the operation of the cleaning apparatus;
figure 8 shows the functional components of the software governing the operation of the cleaning apparatus and the communications between the master and slave processors;
figure 9 shows a flow diagram illustrating the operation of the cleaning apparatus with reference to a single disinfecting unit.

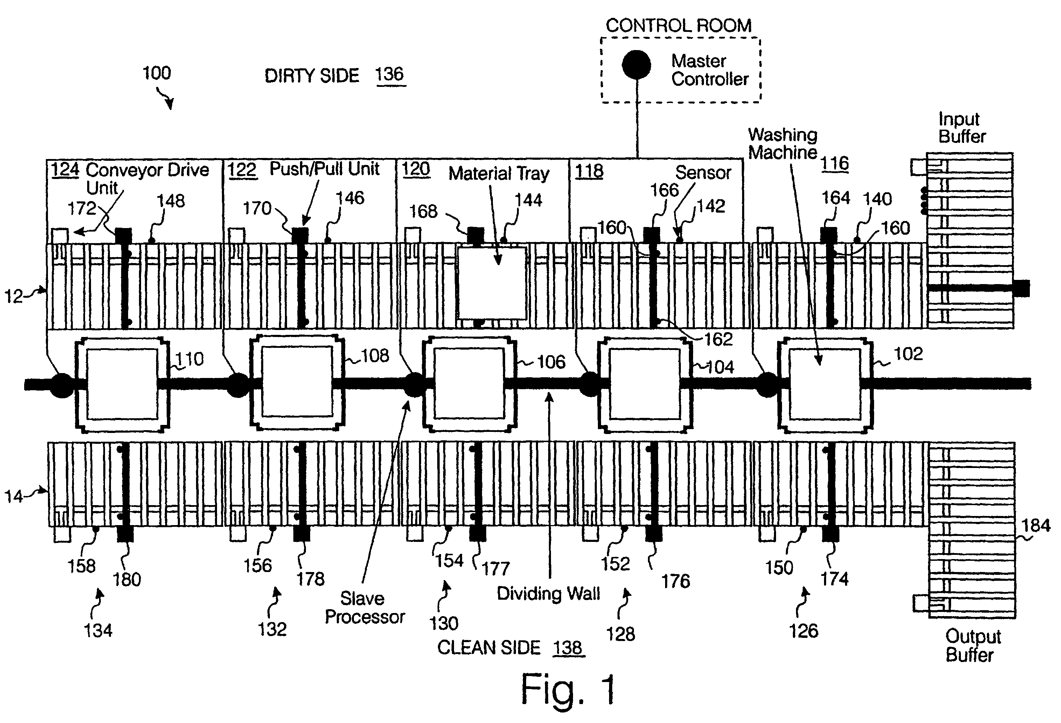

Referring to figure 1, there is schematically illustrated apparatus in accordance with an embodiment of the present invention for cleaning articles comprising a plurality of disinfecting units 102 to 110, an input conveyor 112, an output conveyor 114, a plurality of loading mechanisms 116 to 124 and a plurality of extraction mechanisms 126 to 134. Also shown are the input and output buffers onto and from which the trays are loaded and removed respectively.

The disinfecting units 102 to 110 each comprise two doors; a loading door on the 'dirty' side 136 of the cleaning apparatus 100 and an extraction door which opens onto the 'clean' side 138 of the cleaning apparatus 100. The loading and extraction doors are, in a preferred embodiment, slidably operable and provide for the insertion into or extraction from a cleaning chamber of a tray or basket bearing articles to be cleaned. The articles may comprise, for example, surgical instruments, hollowware, theatre trays, anaesthetic equipment, rigid endoscopes, transit boxes, theatre footwear and laboratory glassware. Any suitable disinfecting unit can be utilised within the cleaning apparatus 100 according to the present invention. Suitable disinfecting units 102 to 110 can be obtained from, for example, Dawson MMP Limited, Waif Road, Elsecar, Barnsley, South Yorkshire, S74 8HJ.

Preferably, the dirty and clean sides of the cleaning apparatus 100 are separated by a screen, for example, a wall. The dirty and clean sides of the apparatus may even open into separate rooms which are linked only by the cleaning chamber of the disinfection units 102 to 110. Such an arrangement advantageously reduces the risk of contamination of clean and sterile instruments by the loading of dirty instruments.

Preferably, a Beta washing disinfector dryer unit available from Dawson MMP Limited is utilised within the present invention. Each disinfecting unit 102 to 110 comprises a slave processor element PE 3 to PE 7 for controlling the operation of the respective disinfecting units 102 to 110.

It can be seen that the cleaning apparatus 100 comprises five modules, module 1 to module 5, each module comprising a disinfecting unit together with appropriate, or portions of the, input and output conveyors as well as respective sensors and loading and extracting mechanisms.

The input conveyor 112 can be any suitable conveyor which can be actuated in a controlled manner, started or stopped, in response to any one of a plurality of control signals. The input conveyor 112 is utilised to accurately position a tray 182 bearing instruments to be cleaned in front of a selectable one of the plurality of disinfecting units 102 to 110.

Similarly, the output conveyor 114 is utilised to carry a tray bearing cleaned or disinfected articles away from a respective disinfecting unit 102 to 110 and ultimately to an output buffer 184 for removal from the cleaning apparatus 100.

It will be appreciated that the conveyors may comprise single dirty and clean side conveyors or that each disinfection unit may comprise a respective conveyor operable independently of the other conveyors of the other disinfection units. Preferably, the present invention utilises respective conveyors which advantageously reduces overall power consumption of the cleaning apparatus 100 as the respective conveyors are only operable when needed as opposed to an single conveyor serving to position a tray before any one of the plurality of disinfecting units. Furthermore, the use of a plurality of smaller conveyors provides for more effective and accurate positioning of the trays, that is to say, improved tray manipulation.

Both the input conveyor 112 and the output conveyor 114 have a plurality of position sensors 140 to 158 for monitoring or determining the position of an article bearing tray with respect to any one of the disinfecting units 102 to 110. The position sensors 140 to 148 are arranged such that a sensor is actuated when the article bearing tray is in the correct position for insertion into a corresponding disinfecting unit. For example, if an article bearing tray 182 is to be loaded into the third disinfecting unit 106, the input conveyor 112 will be operated until position sensor 144 is actuated. The actuation of position sensor 144 will, in turn, halt temporarily the operation of the input conveyor 112 or respective portion thereof.

In a preferred embodiment of the present invention capacitive proximity sensors are utilised as position sensors. Suitable capacitive proximity sensors are available from Farnell and are described in the Farnell electronic component catalogue under model type EC 1808 XPOP available from Farnell.

Although capacitive proximity sensors have been utilised in a preferred embodiment, the present invention is not limited thereto. It will be appreciated by one skilled in the art that other position sensors, for example, inductive proximity detectors, ultrasonic detectors, microswitch sensors, magnetic switch sensors, hall effect devices and LED's together with photo-electric sensors, could alternatively be utilised to implement an embodiment of the present invention.

The capacitive proximity sensors are located such that they can detect the presence of an article bearing tray. Preferably, the capacitive proximity sensors are located on the top of the input or output conveyor walls. Typically, an earthed aluminium tray body at a distance of up to 65 mm or an unearthed aluminium body at a distance of up to 35 mm can be detected, notwithstanding the manufacturer's sensing distance indications of between 2 and 10 mm.

Once a tray bearing instruments to be cleaned is correctly located in front of a selectable one of the plurality of disinfecting units 102 to 110, a corresponding loading mechanism 116 to 124 is utilised, after the door of the corresponding disinfecting unit has been opened, to load the tray into the cleaning chamber of the corresponding disinfecting unit. Each of the loading mechanisms, 116 to 124 can be implemented using a lintra rodless cylinder available from IMI Norgren Limited, Brookside Business Park, Greengate, Middleton, Manchester, M24 1GS. The lintra rodless cylinder comprises a pneumatic cylinder with an internal shuttle linked to an external carriage which can be made to traverse the length of the pneumatic cylinder. The external carriage comprises a protruding arm which is arranged to ensure that the tray is fully inserted into the corresponding disinfecting unit before the slidably closable door thereof is closed.

The sensors of any of the loading mechanisms 116 to 124, such as the first sensor 160 and the second sensor 162 of loading mechanism 116 are used to control or detect the position of external carriage 164, that is to say, these sensors control the supply of the actuating medium, preferably air as it is relatively clean, to the loading mechanism 116.

The loading mechanisms 116 to 124 and the extraction mechanisms 126 to 134 are pneumatically actuated. The pneumatic actuation will be described in detail hereafter.

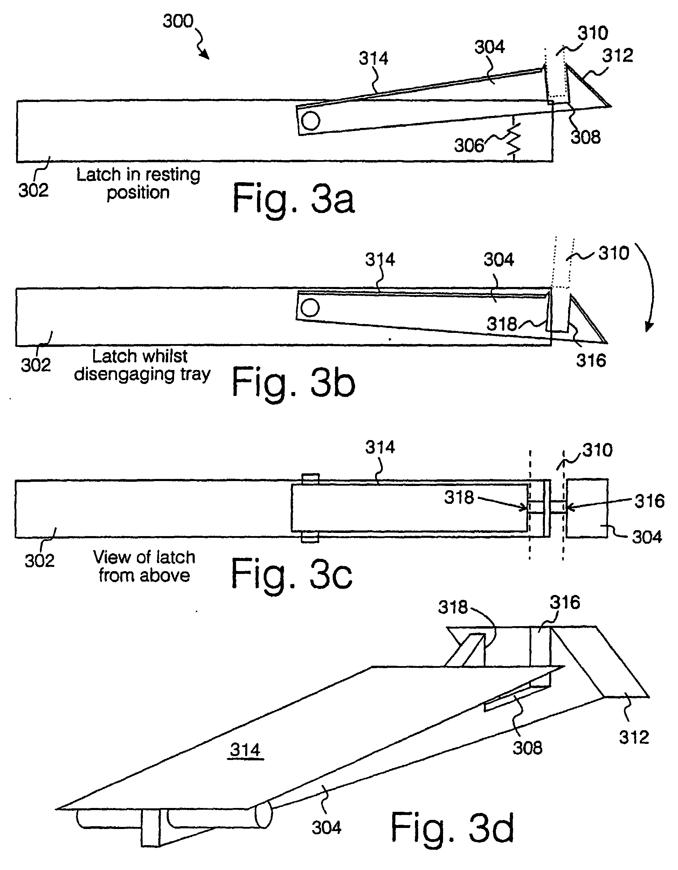

The extraction mechanisms 126 to 134 operate in a substantially similar manner, from both pneumatic actuation and construction view points, to the loading mechanisms 116 to 124. However, whereas the external carriages 164 to 172 of the loading mechanisms 116 to 124 each bear an arm (not shown) for ensuring that the tray is fully disposed within a corresponded disinfecting unit, the external carriages 174 to 180 of extraction mechanisms 126 to 134 each bear an engagement/disengagement mechanism as illustrated in figures 3a to 3d.

With reference to figures 3a to 3d, the engagement/disengagement mechanism 300 comprises a substantially rectangular U-shaped body 302 carrying a pivotally mounted latch 304. The pivotally mounted latch 304 is resiliently biased, via biasing means 306, into an engaging or raised (first) position in which a recess 308 of the latch 304 can receive a portion of the wall 310 of the tray bearing disinfected or cleaned instruments.

In use a first sloping surface 312, upon engagement with the tray wall 310, causes the latch 304 to move progressively from the first position depicted in figure 3a downwardly until the first surface 312 has been fully traversed by tray wall 310 and the tray wall is received into recess 308 as a consequence of the action of the biasing means 306.

Once a portion of the tray wall 310 has been latched or engaged, the external carriage or shuttle 174 to 180 is drawn away from the corresponding disinfecting unit thereby removing the tray bearing cleaned or disinfected instruments from the corresponding disinfecting unit via a second slidably closable door.

The latch 304 also bears a second surface 314 which, upon engagement with a portion of the wall of the output conveyor 314, causes the latch to move from the first position, as depicted in figure 3a, to a second position, as depicted in figure 3b, to thereby disengage the latch or, more particularly, the recess 308, from the corresponding portion of the tray wall 310 and release the tray so that it can be conveyed away from the corresponding disinfecting unit via the output conveyor 114 to the output buffer 184 for collection.

Although the latch 304 depicted in figures 3a to 3d has a rectangularly U-shaped recess 308, the present invention is not limited thereto. An embodiment could equally well be realised in which the side walls 316 and 318 of the recess 308 could progressively diverge from the base of the recess 308. Such an embodiment advantageously provides for the release of the tray in the event that the tray becomes inadvertently fixed or its removal from the disinfecting unit is inadvertently impeded. In such circumstances as the load experienced by the latch 304 is progressively increased, the outwardly divergent wall 316 would be progressively moved towards the disengaged position depicted in figure 3b to thereby release the tray.

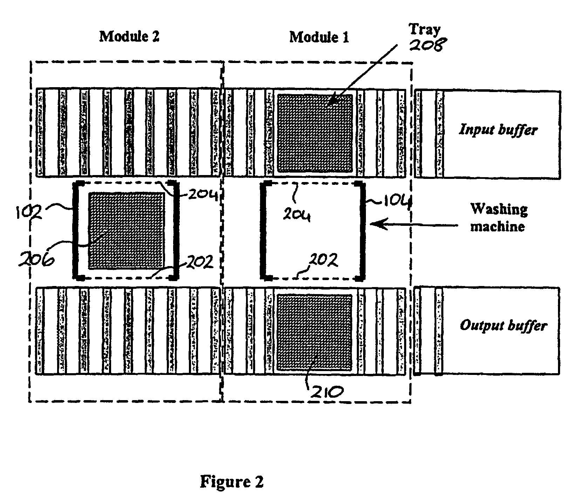

Referring to figure 2, there is shown in the first and second modules of the cleaning apparatus 100 in accordance with an embodiment of the present invention. It can be seen that the disinfecting units 102 and 104 each comprise slidably closable loading doors 202 and corresponding slidably closable extraction doors 204. Disinfecting unit 102 has disposed therein a tray 206 whilst disinfecting unit 104 has had a tray 208 recently removed therefrom and is about to have a further tray 210 inserted thereinto.

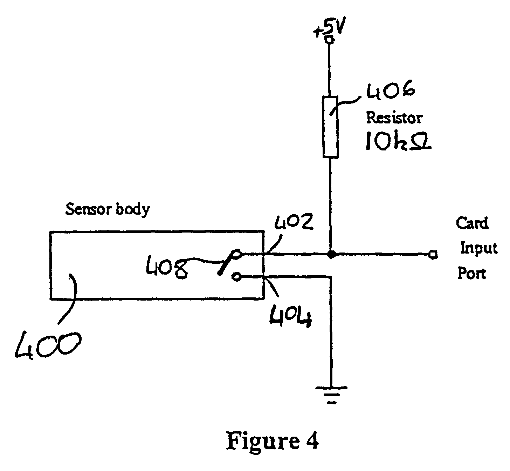

Referring to figure 4, there is schematically depicted a circuit in conjunction with the capacitive proximity sensors. A capacitive proximity sensor 400 is used for each of the position sensors, such as 160 and 162 of the loading mechanisms 116 to 124 and the extraction mechanisms 126 to 134, requires a DC voltage supply of between 10 and 40 volts for the correct operation thereof. Preferably, each capacitive proximity sensor utilises a separate DC voltage supply.

The sensors operate by closing a normally open contact upon sufficient proximity of a tray bearing articles to be cleaned. The capacitive proximity sensor 400 comprises two terminals 402 and 404. The first terminal 402 is connected, via a ten kilo-ohm resistor, to a 5 volt TTL power supply (not shown). The first terminal 402 is also used to provide a control signal indicative of the detection of a tray having sufficient proximity to the capacitive proximity sensor 400. The second terminal 404 is connected to a second voltage level, preferably to ground. Upon detection of a tray, the contact 408 of the capacitive proximity detector 400 is closed thereby pulling the voltage of the first terminal 402 to ground. It will be appreciated that the control signal provided by the first terminal 402 is high or is at 5 volts in the absence of a tray and is low or is at ground in the presence of a tray. This control signal is utilised by the master controller (described hereafter) to control the operation of the input conveyor 112, the output conveyor 114 and the actuation of the loading mechanisms and extraction mechanisms.

The trays bear indicators which provide an indication of the cleaning or disinfection programme to be used in relation to the instruments or articles to be cleaned or disinfected. Referring to figure 1, it can be seen that the input buffer comprises several, four in the present embodiment, capacitive proximity sensors which are used to read the indication of the cleaning programme identified above.

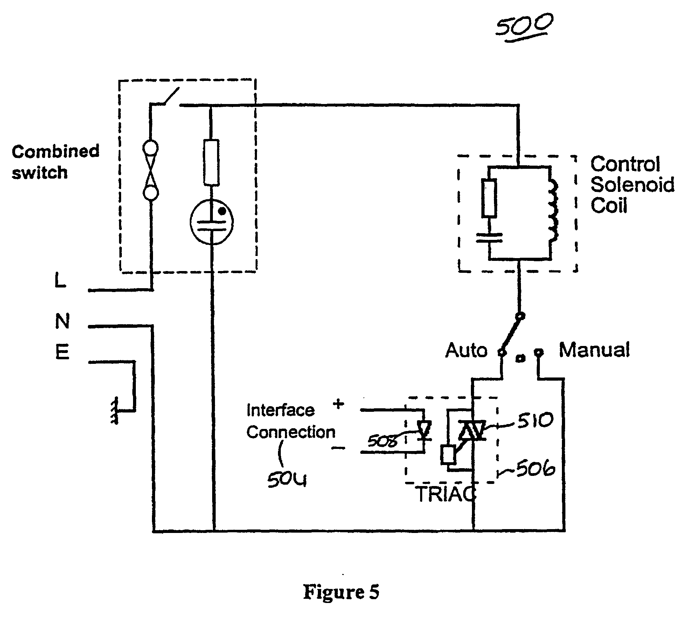

With reference to figure 5, there is schematically shown actuator solenoid circuitry 500 for controlling the operation of the pneumatically actuated loading and extraction mechanisms and, more particularly, the movement of the carriages 164 to 180. V10 nugget 40 solenoids are utilised to control the pneumatic signals to the position actuators 160 and 162 of the loading mechanisms and the corresponding position actuators of the extraction mechanisms. The position actuators require a 240 volt signal to be applied to energise the solenoids 502. The solenoids are energised in response to control signals from a master control unit (described hereafter) via an interface connection 504 used opto isolated triac switches 506.

The triac comprising an LED photo-emitter 508 is connectable to the interface connection 504, a light sensitive triac and a zero crossing circuit 510 and 512 respectively. On receiving a control signal from the interface connection 504, light is generated by and emitted from the LED photo-emitter 508 thereby enabling the triac circuit. However, it should be noted that switching does not occur until the zero cross over circuit has also triggered, that is to say, the zero cross over circuit only activates at the point where the AC wave crosses the zero voltage point. The use of such a zero cross over circuit advantageously reduces noise and the disadvantageous effects of rapidly changing the voltage of a solenoid.

Optionally, the circuit 500 comprises a combined switch incorporating a mains isolation (on/off) switch, safety protection fusing and a power on indicator light. The combined switch is used to protect and isolate the power supply.

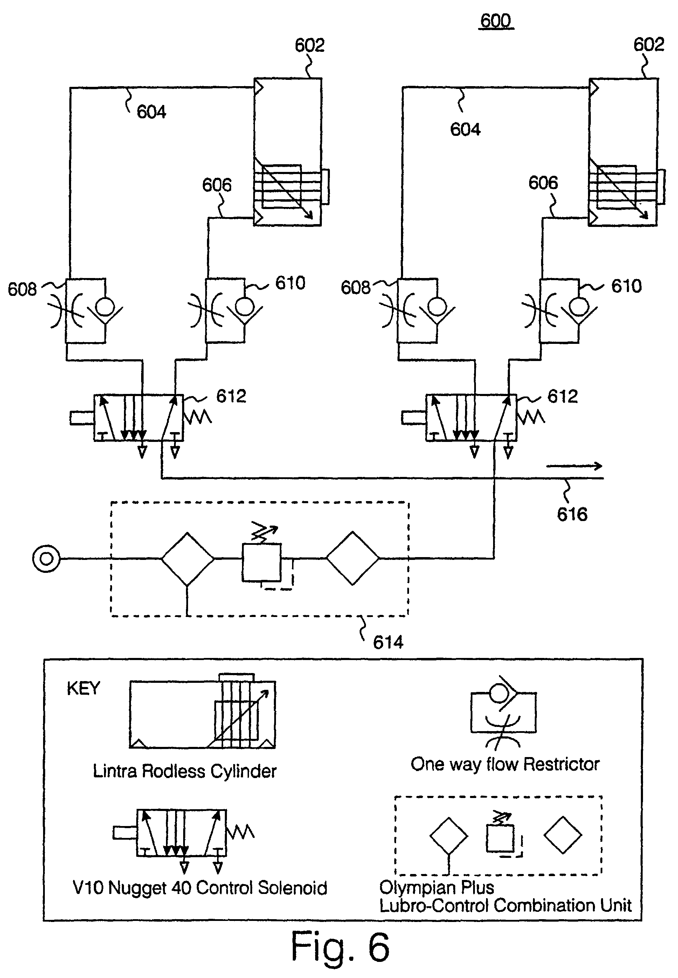

Referring to figure 6, there is shown schematically the pneumatic arrangement 600 for either of the loading mechanism or the extraction mechanism. The pneumatic circuit 600 comprises two lintra rodless cylinders 602 connected via pneumatic supply lines 604 and 606 to respective one way flow restrictors 608 and 610 respectively, the flow restrictors 608 and 610 are connected to V10 nugget 40 control solenoids 612. The V10 nugget 40 control solenoids are used to switch the pneumatic supply so as the enable to the carriages to be movable reciprocally. Each of the V10 nugget 40 control solenoids 612 are connected to an olympian plus lubro-control combination unit 614 which conditions the pneumatic supply.

The pneumatic arrangement depicted relates to a single module. It will be appreciated that each module of the cleaning apparatus has a corresponding pneumatic arrangement. The pneumatic supply line 616 is used to feed the other pneumatic arrangements of the cleaning apparatus.

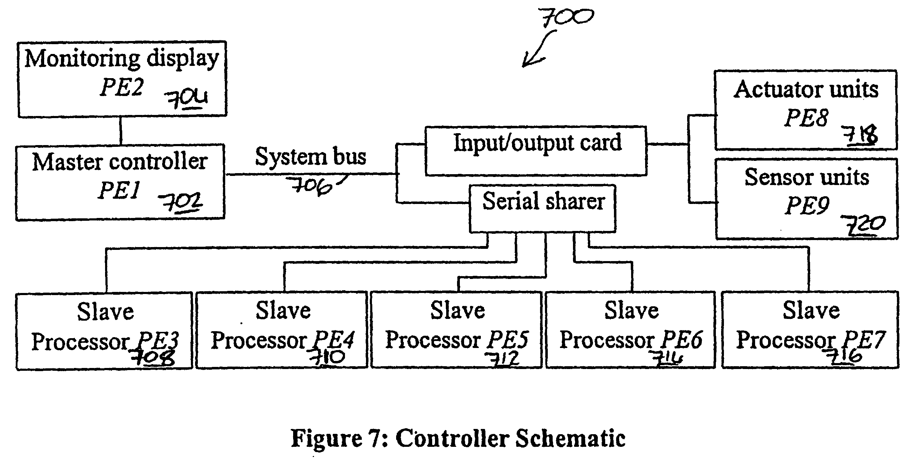

Referring to figure 7, there is shown the inter-processor communication architecture 700 or control system for coordinating and controlling the operation of the cleaning apparatus 100 according to an embodiment of the present invention. The control system comprises a master control or master control processor 702 for controlling the operation of the actuator units and the processor elements within each of the disinfecting or cleaning units 702 to 710, an optional display 704 providing a human interface via which the operation of the communication architecture can be monitored, a system bus 706, a plurality of slave processors, in the instant case five slave processors, one per disinfecting unit. Also attached to the system bus 706 and under control of the master controller 702 are the actuator units 718 and the position sensors 720.

The control system 700 comprises a multipurpose computer together with a plurality of interface or input/output ports as are well understood within the art. The master controller 700 is suitably programmed to be able to communicate with the slave processors 708 to 716 of the disinfection units. The master controller comprises a Microsoft Windows based user interface 704 which provides for the monitoring and controlling of the operation of the disinfection units.

The disinfection units can provide information relating to, for example, the operating temperature of a corresponding disinfection unit. The temperature of a disinfection unit is monitored and recorded periodically during the disinfection cycle. In the event that a disinfection unit is not operating correctly, for example, the cleaning temperature is not sufficiently high to ensure adequate cleaning, the slave processor can inform the master controller. The master controller can then output an indication of the incorrect operation that disinfection unit to the user monitoring the cleaning apparatus.

In the event that the cleaning apparatus repeatedly fails to perform correctly, the master controller can, when scheduling trays for cleaning exclude the failing or failed disinfection unit. This advantageously leads to graceful degradation of the cleaning apparatus.

The master controller comprises a plurality of input/output cards for communication with the slave processors and for detection of the signals produced by the position and actuator sensors. Communication with the plurality of slave processors is by way of an SS10 serial sharer as is well known within the art. Any suitable communication protocol can be used to support the communication between the master and slave processors.

The actuator units 718 are utilised to control the input conveyor 112, the output conveyor 114, more particularly the carriages 164 to 172 of the loading mechanisms and the output mechanisms 126 to 134, more particularly the extractor carriages 174 to 180 of the output mechanisms.

The master controller and slave processors can be realised using any suitably programmed processor. The control system including master controller 702 and slave processors can alternatively be realised using embedded processors or programmable gate arrays suitably configured to replace the microcomputer.

Once a tray is loaded onto the input buffer, the indicator means is read by the four capacitive sensors to determine the appropriate disinfection programme. The data or signals obtained from the capacitive sensors is transmitted to the master controller 702. The master controller 702 upon receipt of the programme data, downloads to a selectable or available one of the slave processors, a program suitable for implementing the appropriate cleaning programme.

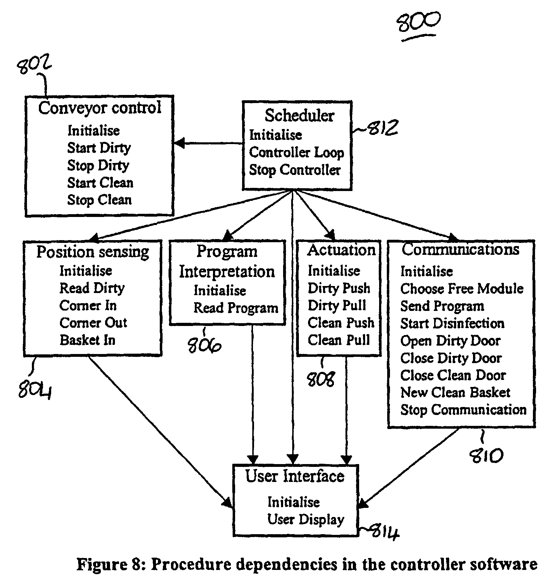

Referring to figure 8, there is shown the functional components 800 of the software controlling the master controller 700. Seven functional modules are provided for the overall control of the cleaning apparatus 100. All modules comprise software for initialising a corresponding part of the cleaning apparatus 100.

A conveyor module 802 which allows the master controller to start and stop the transport mechanism or input 112 and output 114 conveyors. The conveyor module 802 also comprises software for initialising the conveyors.

A position sensor module 804 is used to monitor the position sensors. Corner In and Corner out are used to read the sensors of the input and output buffers respectively, basket in is used to determine whether or not the tray is safely disposed within a disinfecting unit.

A Program Interpretation module 806 is used to read the cleaning programme shown on the side of a tray, that is, indicated by the indicator means.

An actuation module 808 is utilised to control the loading and extraction mechanisms. Dirty push and pull are commands to the pneumatics for moving a tray into or out of a disinfection unit respectively. Similarly for the corresponding clean push/pull commands.

A communication module 810 is provided to give effect to communication between the master and slave processors. The choose free module function selects one of the plurality of disinfection units to be used to clean the next tray to be cleaned. The send program module selects and downloads a program to be used by the slave processor in providing the cleaning programme in accordance with the programme indicated by the indicator means. The Start Disinfection command is used to commence the disinfection operation of the disinfection unit. The open and close door commands are used to open and close the dirty and clean side doors. Finally, a Stop Communication command is used to terminate communication with the slave processor.

A scheduler module 812 is used to schedule the cleaning operations of the cleaning apparatus. The scheduler governs the overall interaction between the various software modules. The schedule module of the software is used to decide which tray should be cleaning using which disinfection unit and at what time.

A user interface module 814 is used to provide information to the user or human operator of the cleaning apparatus. Typically, the human operator will be remotely situated from the cleaning apparatus; remote including being physically separated or screened from the cleaning apparatus or situated in a different room to the cleaning apparatus. The separation of the human operator from the environment containing the disinfection units, together with potentially hazardous cleaning fluids, represents an improvement in the safety of the operating conditions of the human operators.

The control software functionally depicted in figure 8 utilises signals from the various sensors, actuators and disinfection units to determine which disinfection unit should be used for cleaning the next dirty tray. The entire cleaning operation from when a tray bearing dirty articles in placed onto the input buffer until the same tray is removed from the output buffer, including the detection of the appropriate cleaning programme, disinfection unit initialisation, tray manipulation and environment monitoring, is controlled by the master controller 702.

The master control unit can periodically interrogate the slave processors to determine the current status of their cleaning programme. The results of the determination can be used to schedule the cleaning of the instruments. Alternatively, the slave processors may periodically interrupt the master processor to inform the latter of the current stage of a cleaning or disinfection cycle.

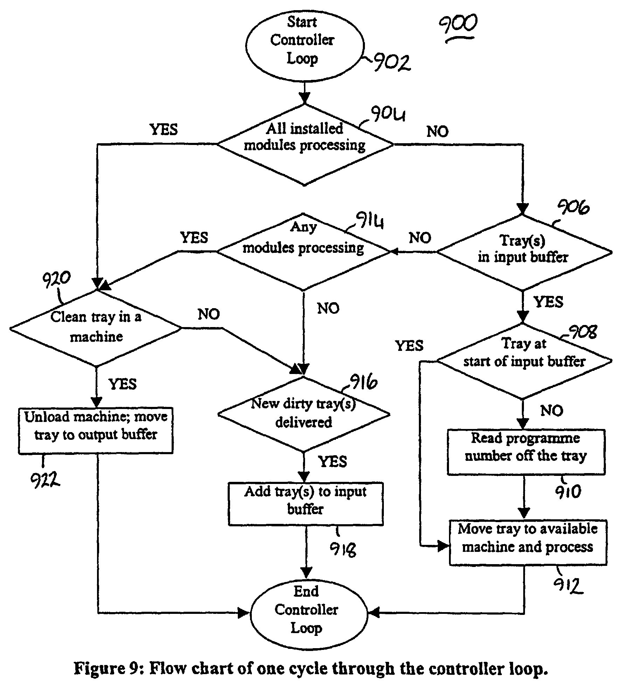

Referring to figure 9, there is shown a flowchart 900 illustrating the overall control of the master controller 702. The master controller is initialised and commences processing at step 902. A determination is made at step 904 as to whether or not any of the modules or disinfection units are free for use. If a disinfection module or unit is free, step 906 determines whether or not there are any trays loaded in the input buffer ready for cleaning. If a tray is in the input buffer, step 908 determines whether or not the tray is at the start of the input buffer, and if not, the cleaning programme indicated by the indicator means is read by the master controller at step 910. If the tray was not at the start of the input buffer or after the cleaning programme has been read by the master controller, the tray is moved to an available disinfection unit at step 912.

In the event that there are no trays in the input buffer, control passes from step 906 to step 914, where a determination is made as to whether or not any of the disinfection units are currently performing cleaning operations at step 914. If none of the disinfection units are currently cleaning trays, a determination is made at step 916 as to whether or not any new trays containing instruments to be cleaned, that is to say, dirty trays, have been delivered for cleaning. This step continues in a loop until such a dirty tray does arrive whereupon the tray is transferred to the input buffer at step 918.

If all or any of the disinfection units are currently being utilised, step 920 is executed. A determination is made at step 920 as to whether or not there are any trays or instruments which have been cleaned and require to be removed from the disinfection units. If there are no clean trays in the disinfection units, control passes to step 916. If there are clean trays in the disinfection units, control passes to step 922 where the tray of clean or disinfected articles is removed from the corresponding machine.

A module comprises all of the hardware and control software associated with any given disinfection unit. For example, module 1 comprises disinfection unit 102, dirty and clean transport or conveyor means, dirty positions actuator sensors 160 and 162, actuator carriage 164, a slave processor and capacitive proximity sensor 140.

The reader's attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

## Claims

1. Apparatus (100) for cleaning articles comprising a plurality of cleaning units (102; 104; 106; 108; 110) each having a chamber for receiving a tray loaded with articles to be cleaned, said cleaning units (102; 104; 106; 108; 110) being arranged to clean said articles, a transport mechanism (112,114) for loading and removing said tray of articles into and out of selectable ones of said chambers, and control means for automatically coordinating the operation of said plurality of cleaning units and said transport mechanism, wherein said control means comprises a master control processor (702) and associated with each of said cleaning units at least one slave processor (708; 710; 712; 714; 716) for controlling the operation of said cleaning units in response to data received from the master control processor (702).

2. Apparatus as claimed in claim 1, wherein said chamber comprises at least a first slidably closable door via which the tray can be received into or removed from the chamber.

3. Apparatus as claimed in claim 2, further comprising a second slidably closable door via which the tray can be received into or removed from the chamber.

4. Apparatus as claimed in claim 3, wherein said first and second slidably closable doors are disposed on substantially opposite sides of the cleaning apparatus.

5. Apparatus as claimed in any preceding claim, wherein said transport means comprises a conveyor for conveying trays loaded thereon to a first position for loading into said chamber.

6. Apparatus as claimed in claim 5, wherein said first position is selectable by said control means in response to at least one of a plurality of conditions.

7. Apparatus as claimed in claim 6, wherein one of said plurality of conditions is governed by a current utilisation status of a cleaning unit and, preferably, wherein one of said plurality of conditions is governed by the remaining time until the end of the operation of a currently executing cleaning cycle of the cleaning unit.

8. Apparatus as claimed in any preceding claim, wherein said transport mechanism is either operable vertically or horizontally.

9. Apparatus as claimed in any preceding claim, wherein said transport mechanism comprises an input mechanism for correctly positioning the tray in front of a selectable one of said plurality of cleaning units for loading thereinto and an output mechanism for transporting said tray away from a respective cleaning unit and, preferably, wherein said input and output transport mechanisms are disposed on substantially opposite sides of said apparatus.

10. Apparatus as claimed in any preceding claim, wherein said tray bears indicator means for providing an indication of at least one of either the type of article to be cleaned or the cleaning programme to be used in cleaning;
and/or wherein said first control processor is remotely disposed, screened or physically separated from said cleaning units and said transport mechanism;
and/or wherein said transport mechanism comprises a plurality of separately operable conveyors, one per cleaning unit.

11. Apparatus as claimed in any preceding claim, wherein said control means further comprises means for collating and outputting, via a user interface, data related to the operating conditions of the cleaning units.

## Revendications

1. Appareil (100) de nettoyage d'articles, comprenant une pluralité d'unités de nettoyage (102 ; 104 ; 106 ; 108 ; 110), ayant chacune une chambre pour recevoir un plateau chargé des articles devant être nettoyés, lesdites unités de nettoyage (102 ; 104 ; 106 ; 108 ; 110) étant agencées pour nettoyer lesdits articles, un mécanisme de transport (112, 114) pour charger et retirer ledit plateau d'articles dans et hors de celles sélectionnables desdites chambres, et un moyen de commande pour coordonner automatiquement le fonctionnement de ladite pluralité d'unités de nettoyage et dudit mécanisme de transport, dans lequel ledit moyen de commande comprend un processeur de commande pilote (702) et, associé avec chacune desdites unités de nettoyage, au moins un processeur auxiliaire (708 ; 710 ; 712 ; 714 ; 716) pour commander le fonctionnement desdites unités de nettoyage en réponse à des données reçues du processeur de commande pilote (702).

2. Appareil selon la revendication 1, dans lequel ladite chambre comprend au moins une première porte susceptible fermée par coulissement par l'intermédiaire de laquelle le plateau peut être reçu dans ou retiré de la chambre.

3. Appareil selon la revendication 2,comprenant en outre une seconde porte susceptible d'être fermée par coulissement par l'intermédiaire de laquelle le plateau. peut être reçu dans ou retiré de la chambre.

4. Appareil selon la revendication 3, dans lequel lesdites première et seconde portes susceptibles d'être fermées par coulissement sont disposées sur des côtés sensiblement opposés de l'appareil de nettoyage.

5. Appareil selon l'une quelconque des revendications précédentes, dans lequel ledit moyen de transport comprend une installation de transport pour transporter des plateaux chargés sur celui-ci jusque dans une première position pour le chargement dans ladite chambre.

6. Appareil selon la revendication 5, dans lequel ladite première position est sélectionnable par ledit moyen de commande en réponse à au moins l'une d'une pluralité de conditions.

7. Appareil selon la revendication 6, dans lequel l'une de ladite pluralité de conditions est gouvernée par un état d'utilisation courant d'une unité de nettoyage et, de préférence, dans lequel l'une de ladite pluralité de conditions est gouvernée par le temps restant jusqu'à la fin du fonctionnement d'un cycle de nettoyage s'exécutant de façon courante de l'unité de nettoyage.

8. Appareil selon l'une quelconque des revendications précédentes, dans lequel ledit mécanisme de transport est actionnable ou bien verticalement ou bien horizontalement.

9. Appareil selon l'une quelconque des revendications précédentes, dans lequel ledit mécanisme de transport comprend un mécanisme d'entrée pour positionner de façon correcte le plateau devant l'une sélectionnable de ladite pluralité d'unités de nettoyage pour le chargement dans celle-ci et un mécanisme de sortie pour transporter ledit plateau hors d'une unité de nettoyage respective et, de préférence, dans lequel lesdits mécanismes de transport d'entrée et de sortie sont disposés sur des côtés sensiblement opposés dudit appareil.

10. Appareil selon l'une quelconque des revendications précédentes, dans lequel ledit plateau porte des moyens indicateurs pour fournir une indication d'au moins l'un soit du type d'article devant être nettoyé soit du programme de nettoyage devant être utilisé dans le nettoyage ;
et/ou dans lequel ledit premier processeur de commande est disposé de façon éloignée, protégé par écran ou physiquement séparé desdites unités de nettoyage et dudit mécanisme de transport ;
et/ou dans lequel ledit mécanisme de transport comprend une pluralité d'installations de transport actionnables de façon séparée, une par unité de nettoyage.

11. Appareil selon l'une quelconque des revendications précédentes, dans lequel ledit moyen de commande comprend en outre un moyen pour compiler et sortir, par l'intermédiaire d'une interface utilisateur, des données se rapportant aux conditions de fonctionnement des unités de nettoyage.

## Patentansprüche

1. Vorrichtung (100) zum Reinigen von Gegenständen, mit einer Vielzahl von Reinigungseinheiten (102; 104; 106; 108; 110), von denen jede eine Kammer zum Aufnehmen einer Schale hat, die mit zu reinigenden Gegenständen gefüllt ist, wobei die Reinigungseinheiten (102; 104; 106; 108; 110) dazu ausgestaltet sind, um die Gegenstände zu reinigen, einem Transportmechanismus (112, 114) zum Einladen und Ausladen der Schale mit Gegenständen in und aus auswählbaren Kammern und einer Steuereinrichtung zum automatischen Koordinieren des Betriebs der Vielzahl von Reinigungseinheiten und des Transportmechanismus, wobei die Steuereinrichtung einen Master-Steuerprozessor (702) aufweist und wobei zumindest ein Slave-Prozessor (708; 710; 712; 714; 716) vorgesehen ist, der mit jeder der Reinigungseinheiten in Beziehung steht, um den Betrieb der Reinigungseinheiten in Reaktion auf Daten zu steuern, die von dem Master-Steuerprozessor (702) empfangen werden.

2. Vorrichtung nach Anspruch 1, bei der die Kammer zumindest eine erste verschiebbar verschließbare Tür aufweist, über die die Schale in die Kammer eingeladen und aus dieser ausgeladen werden kann.

3. Vorrichtung nach Anspruch 2, außerdem mit einer zweiten verschiebbar verschließbaren Tür, über die die Schale in die Kammer eingeladen oder daraus ausgeladen werden kann.

4. Vorrichtung nach Anspruch 3, bei der die erste und die zweite verschiebbar verschließbare Tür im wesentlichen an gegenüberliegenden Seiten der Reinigungsvorrichtung angeordnet sind.

5. Vorrichtung nach einem der vorhergehenden Ansprüche, bei der die Transporteinrichtung eine Fördereinrichtung aufweist, um darauf angeordnete Schalen zu einer ersten Position zu fördern, um in die Kammer eingeladen zu werden.

6. Vorrichtung nach Anspruch 5, bei der die erste Position durch die steuereinrichtung in Reaktion auf zumindest einen von einer Vielzahl von Zuständen auswählbar ist.

7. Vorrichtung nach Anspruch 6, bei der einer dieser Vielzahl von Zuständen durch einen aktuellen Verwendungszustand einer Reinigungseinheit bestimmt ist und, vorzugsweise, bei der einer der Vielzahl von Zuständen durch die verbleibende Zeit bis zum Ende des Betriebs eines aktuell ausgeführten Reinigungszyklus der Reinigungseinheit bestimmt ist.

8. Vorrichtung nach einem der vorhergehenden Ansprüche, bei der der Transportmechanismus entweder vertikal oder horizontal betrieben werden kann.

9. Vorrichtung nach einem der vorhergehenden Ansprüche, bei der der Transportmechanismus einen Eingabe-Mechanismus zur korrekten Positionierung der Schale vor einer ausgewählten der Vielzahl von Reinigungseinheiten, um darin eingeladen zu werden, und einen Ausgabe-Mechanismus aufweist, um die Schale von einer jeweiligen Reinigungseinheit wegzutransportieren, und bei der die Eingabe- und der Ausgabe-Transportmechanismen vorzugsweise an im wesentlichen gegenüberliegenden Seiten der Vorrichtung angeordnet sind.

10. Vorrichtung nach einem der vorhergehenden Ansprüche, bei der die Wanne Indikator-Einrichtungen trägt, um eine Angabe von dem Typ des zu reinigenden Gegenstandes und/oder des Reinigungsprogrammes anzugeben, das zum Reinigen verwendet wird;
und/oder bei der der erste Steuerprozessor entfernt angeordnet, abgeschirmt oder physikalisch von den Reinigungseinheiten und dem Transportmechanismus getrennt ist;
und/oder bei der der Transportmechanismus eine Vielzahl von separat betätigbaren Förderern aufweist, und zwar einer pro Reinigungseinheit.

11. Vorrichtung nach einem der vorhergehenden Ansprüche, bei der die Steuereinrichtung außerdem Einrichtungen zum Ordnen und Ausgeben, über eine Benutzerschnittstelle, von Daten aufweist, die mit den Betriebszuständen der Reinigungseinheiten in Beziehung stehen.
